# EUROPEAN PATENT APPLICATION

(11) **EP 3 578 149 A1**
(43) Date of publication of application: **11.12.2019**
(21) Application number: 19177850.5
(22) Date of filing: 03.06.2019
(51) Int. Cl.: A61F 13/15, B65H 54/28

(54) **A GUIDING DEVICE OF AN APPARATUS FOR PRODUCING SANITARY ARTICLES FOR APPLYING A WIRE AND RELATIVE METHOD**

(30) Priority: 08.06.2018 IT 201800006019
(71) Applicant: Fameccanica.Data S.p.A., 65129 Pescara (IT)
(72) Inventor: ANTONELLI, Gabriele Biagio, 67100 Poggio Santa Maria (L'Aquila) (IT); CENTORAME, Oscar, 64021 Giulianova (Teramo) (IT)
(74) Representative: Marchitelli, Mauro

(57) **Abstract**

A guiding device (10) of an apparatus for producing sanitary articles for applying at least one thread (11) onto a substrate (12) intended for producing sanitary articles, wherein said guiding device (10) is able to guide the thread (11) quickly, accurately, and stably without being subject to wear and contamination problems, while ensuring reduced energy consumption. The present invention also relates to a method for applying a thread (11) onto a substrate (12) intended to produce sanitary articles.

## Description

### FIELD OF APPLICATION

Embodiments of the present invention relate to a guiding device of an apparatus for producing sanitary articles for applying a thread onto a substrate intended for producing these sanitary articles such as, for example, diapers, pull-ups, incontinence pads, sanitary napkins, or other articles designed to absorb body fluids.

Embodiments of the present invention relate to a guiding device of an apparatus for producing sanitary articles configured to apply elastic threads onto a substrate such as, for example, a continuous web material, a continuous composite sheet, or a discrete component.

Embodiments of the present invention also relate to a method utilizing one or more guiding devices of an apparatus for producing sanitary articles for applying threads onto a substrate intended for producing sanitary articles.

### STATE OF THE ART

There are sanitary articles made with non-elastic materials which, in relation to the specific articles to be made and to the desired wearability to be achieved, can be rendered elastic, for example, by trapping elastic threads between them.

A typical example of applying elastic threads to a diaper is that of the waist band, or of the leg cuffs, in which pleated regions are made by means of these elastic threads, which provide a certain extensibility to the material.

Entrapment of pre-tensioned elastic threads between two sheets of non-elastic materials for example a non-woven fabric, is known by locally fixing the sheets together and/or the elastic threads tensioned to the sheets so as to locally constrain the elastic threads along a pattern of entrapment.

In relation to the specific entrapment pattern and the elastic action to be provided to the specific area of the article, the elastic thread is guided along a specific path adjusting the feeding speed during its application onto the substrate.

Some guiding devices comprise a linear guide defining a linear direction and a carriage provided with a thread-carrying tool which is configured to move on the linear guide in relation to the specific entrapment pattern to be achieved.

During the movement of the carriage on the linear guide, the carriage makes multiple changes of direction that significantly wear out both the wheels, or balls, of the carriage, and the surface of the linear guide, requiring frequent maintenance and/or lubrication.

Due to the wear of the balls of the carriage and the surface of the linear guide, the thread is typically supplied to the substrate incorrectly, making the resulting sanitary articles non-conformant to the required specifications.

Solutions are known that use magnetic actuators to speed up the movement of the carriage provided with balls which, however, are still unsuitable for the specific application, since the problems relating to wear are accentuated.

These known solutions have not provided satisfactory results in relation to the high standards of stability and precision required and, furthermore, are unsuitable in contexts subject to contamination by cellulose or other fibrous and/or dusty materials.

It is known that these contaminants are moved during movements of the carriage on the linear guide and are deposited on the latter, making translation of the carriage more difficult, and risking compromising the operation of the system.

This problem is further emphasized by the presence of the lubricant which acts as a glue for dust and which, when contaminated, loses its lubricating action, making the surface on which the carriage slides unsuitable for sliding.

Known solutions have been developed in fields that are significantly different from that of the present invention which, in their design and for the performances they are able to achieve, cannot be used in an apparatus for making sanitary articles.

Document EP-A-1.148.016 (EP'016) describes an apparatus for winding a thread onto a reel, in which the apparatus consists of a carriage movable on a magnetic guide and is configured to reach speeds from 2 m/s to 6 m/s.

Document EP'016 describes and addresses the problem of changing direction of the movable carriage and, in particular, proposes the use of a control unit configured for controlling the speed of the movable carriage during the inversion.

It is known that the reel winding systems have problems mainly related to the winding in succession of the turns which, if incorrectly overlapped, can make the reel non-homogeneous.

The apparatuses for winding thread on reels envisage that the movable carriage can perform repeated movements along a linear direction between two fixed and spaced-apart end positions of a limited length.

In the field of apparatuses for producing sanitary articles, it is known that the thread-guiding devices must convey the threads so as to produce various and different entrapment patterns quickly in order to produce different formats of sanitary articles.

In the field of sanitary articles, it is required that the movable carriage is moved with a linear stroke even greater than 30 cm and with speeds and accelerations equal to if not greater than those of the field of apparatus for producing reels.

Document DE-A-10.2013.225653 (DE'653) describes an apparatus for winding a thread onto a reel consisting of a movable carriage coupled to a magnetic beam.

The movable carriage is kept spaced apart from the magnetic beam by means of a permanent magnetic field obtained by the interaction between a plurality of permanent magnets arranged on the movable carriage and the magnetic beam itself.

This known solution envisages that the movable carriage can move passively along the longitudinal extension in relation to the position of the thread.

The apparatus described in DE'653 also comprises aerostatic brakes placed at the ends of the magnetic beam and oriented towards the movable carriage. These aerostatic brakes are configured to slow down the movable carriage when the carriage reaches the ends so that it does not hit against the ends.

DE'653 also describes the possibility of using aerostatic brakes as a means of moving the movable carriage which, under the thrusting action of compressed air jets from the aerostatic brakes, can move along the magnetic beam.

This known solution has considerable problems in that it is not possible to precisely control the movable carriage and, moreover, it is not possible to move the movable carriage in order to obtain varied and different entrapment patterns of the threads between the sheets with the speeds required in the field of sanitary articles.

Document WO-A-00/24663 (WO'663) describes a system for conveying a thread towards a reel provided with a rotating element configured to feed the thread rotating about an axis of rotation defined by a pin to which it is connected.

The rotating element of WO'663 is positioned spaced apart from a plane and is moved by means of a magnetic movement assembly. The rotating element is provided with an inductive circuit which, when powered, couples with the magnetic field generated by magnets installed on the plane.

In addition to the problems of the prior art already described, this known solution has the further problem that the end that guides the thread towards the reel follows a curvilinear path and, even if the rotating element is spaced apart from the plane, it can bend towards the plane in relation to the direction of the thread and its elasticity.

This last known solution cannot be used in an apparatus for producing sanitary articles since the performances required in the field cannot be satisfied by it.

The problems that an expert in the field of apparatuses for producing sanitary articles must confront and, in particular, those relating to the high speed of movement of the thread-guide, as well as those relating to the possibility of producing varied and different patterns of entrapment of the threads between two sheets, are neither described nor addressed by known solutions.

The known solutions cannot be used in apparatuses for producing sanitary articles that require speeds of up to 10 m/s, with accelerations at full speed even higher than 230 m/s² and with strokes even greater than 30 cm.

These known solutions do not allow sanitary articles that have sheets between which elastic threads are trapped (and with the desired pattern of entrapment of the threads between the sheets) to be produced quickly, in which the positioning error of the thread along the desired entrapment pattern does not exceed 5 mm.

In this context, the need has arisen to provide a guiding device of an apparatus for producing sanitary articles which, in addition to ensuring high standards of stability, precision and performance, is able to overcome the problems connected to contamination and those related to wear of the sliding parts.

There is therefore the need to improve and make a guiding device available of an apparatus for producing sanitary articles to apply threads onto a substrate intended for producing these sanitary articles, which overcomes at least one of the drawbacks of the prior art.

One object of the present invention is to provide a guiding device capable of overcoming the problems related to wear, which does not require frequent maintenance and lubrication, and which - at the same time - allows the thread to be supplied on the substrate in a reproducible and precise manner.

Another object of the present invention is to provide a guiding device capable of supplying the thread in a stable, controlled and high-speed manner.

Another object of the present invention is to provide a guiding device capable of overcoming the problems related to contamination, while eliminating the problems relating to wear and lubrication.

It is a further object of the present invention to provide a method using one or more guiding devices for applying threads onto a substrate intended for producing sanitary articles that is able to overcome at least one of the problems of the prior art.

The present Applicant has devised, tested and embodied this invention to overcome the shortcomings of the prior art and to obtain these and additional objects and advantages.

### SUMMARY OF THE INVENTION

The present invention is expressed and characterized in the independent claims, while the dependent claims set out other characteristics of the present invention or variants of the idea of the main solution.

In accordance with the aforesaid objects, the present invention relates to a guiding device of an apparatus for producing sanitary articles for applying at least one thread onto a substrate intended to produce these sanitary articles, which comprises a first body movable along a linear direction and configured to guide the thread, and at least one second body fixed with respect to the first body. For example, the substrate may comprise a continuous web or other material.

The guiding device also comprises a magnetic movement assembly associated with the first body, and with the at least one second body, for moving the first body along the linear direction by means of a magnetic force. Thanks to the action of a spacer assembly, a gap is formed between the first body and the at least one second body, also considering any elements associated with them.

The magnetic movement assembly comprises a plurality of magnets, placed on the first body, and means for generating a variable magnetic field, placed on the at least one second body, in which the variable magnetic field is such as to cooperate, in use, with at least part of the magnets to move the first body along the linear direction by magnetic force.

In accordance with an aspect of the present invention, the guiding device comprises a spacer assembly configured to space the first body apart from the at least one second body to form a gap between them by means of an air cushion.

Thanks to the presence of an air cushion that acts as a barrier for possible contaminants, this solution hinders the access of the cellulose fibers and/or other contaminants into the gap, as well as their deposit on the surfaces defining the gap.

This solution does not require the use of lubricants and, furthermore, significantly reduces the wear of the moving parts, since there are no rolling elements such as, for example, balls, rollers, or other elements, in contact with the first body and/or with the at least one second body.

The presence of the air cushion also makes it possible to further speed up the movement of the first body along the linear direction, while at the same time ensuring high stability and precision to apply the thread onto the substrate correctly.

Both when the spacer assembly is active and when it is deactivated, or when the first body is stationary with respect to the at least one second body, there is no contamination of the surfaces defining the gap. In fact, in the first case the spacer assembly prevents contaminants from entering the gap, while in the second case, since the bodies are in mutual contact, the gap does not exist or is significantly reduced.

According to possible embodiments, the spacer assembly may comprise a plurality of aerostatic spacer elements each configured to generate a respective air cushion in order to locally distance the first body from the at least one second body.

In accordance with possible embodiments, the aerostatic spacer elements can be placed on the at least one second body on the sides of the magnetic movement unit.

According to possible embodiments, the spacer assembly may comprise at least one magnetic spacer element configured to locally distance the first body from the at least one second body by means of another magnetic force.

In accordance with possible embodiments, the guiding device may comprise at least two second bodies between which the first body is interposed, wherein each of the at least two second bodies is provided with respective means for generating a respective variable magnetic field.

According to possible embodiments, the ratio between the respective lengths along the linear direction of the first body and of the second body can be greater than 1.

According to possible embodiments, the guiding device may comprise at least one management and control unit configured to modulate the variable magnetic field generated by the means, by means of a control signal sent by the management and control unit to the means.

According to possible embodiments, the guiding device may comprise at least two management and control units, each configured to modulate the respective variable magnetic field generated by the respective means of the at least two second bodies, by means of a respective control signal sent by the management and control unit to the respective means.

In agreement with possible embodiments, the guiding device may comprise a support member connected to the first body and configured to connect a tool to carry the thread, in which the support member extends, at least in part, out of the at least a second body.

According to possible embodiments, the guiding device may comprise a cooling unit configured to cool at least the first body by means of a cooling fluid.

In accordance with possible embodiments, the present invention also relates to a method for applying a thread onto a substrate intended to produce sanitary articles.

In accordance with one aspect of the present invention, the method envisages: associating the thread with a first body movable along a linear direction; spacing the first body apart from the at least one second body fixed with respect to the first body by means of an air cushion; and moving the first body along the linear direction by a magnetic force so as to guide the thread to apply it on the substrate.

According to possible embodiments, the movement of the first body is obtained by means of a magnetic movement assembly comprising a plurality of magnets, placed on the first body, and means for generating a variable magnetic field, placed on the at least one second body.

In accordance with possible embodiments, the method may envisage generating electrical energy by electromagnetic induction, while the first body decelerates, and feeding an apparatus with said generated electric energy.

According to possible embodiments, the method may envisage setting a desired timetable defining the position of the first body along the linear direction in relation to time, and for moving the first body according to the set timetable.

In accordance with possible embodiments, the method may envisage modulating the movement of the first body in relation to the elasticity of the thread and to the instantaneous position of the first body along the linear direction.

### ILLUSTRATION OF THE DRAWINGS

These and other characteristics of the present invention will become clear from the following description of embodiments, given as a non-limiting example, with reference to the attached drawings wherein:
- Figure 1 is a perspective view of a guiding device of an apparatus for producing sanitary articles according to a possible embodiment of the present invention;
- Figures 2a and 2b are two side views of two guiding devices of an apparatus for producing sanitary articles according to possible embodiments of the present invention;

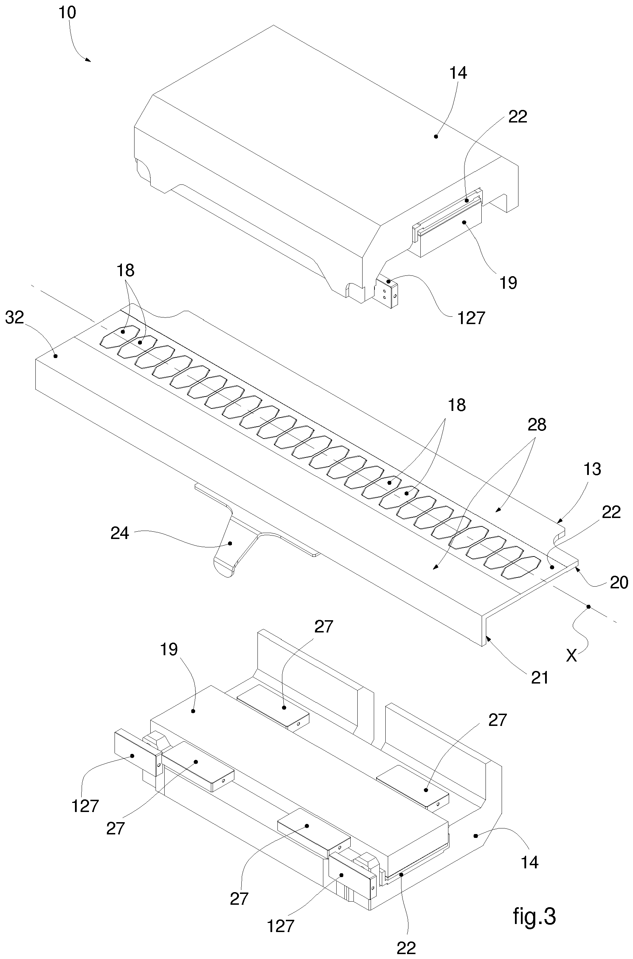

- Figure 3 is an exploded view of Figure 1;

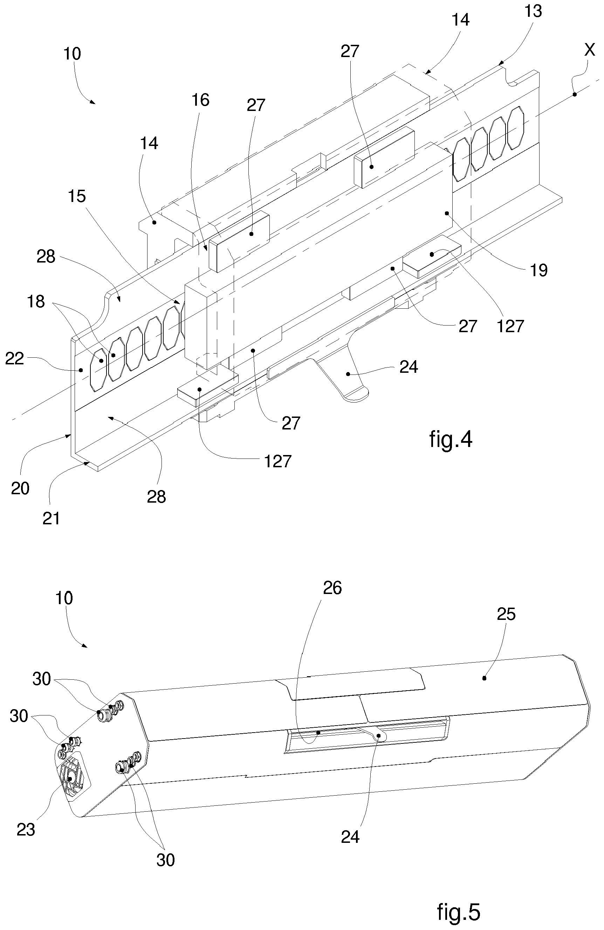

- Figure 4 is a schematic perspective view of a guiding device of an apparatus for producing sanitary articles according to a possible embodiment of the present invention;
- Figure 5 is a perspective view of a guiding device of an apparatus for producing sanitary articles according to a possible embodiment of the present invention.

To facilitate understanding, identical reference numbers have been used, where possible, to identify identical common elements in the Figures. It should be understood that elements and characteristics of an embodiment can be conveniently incorporated in other embodiments without further specification.

### DESCRIPTION OF THE EMBODIMENTS

Embodiments described herein, with reference to the figures, refer to a guiding device 10 of an apparatus for producing sanitary articles for applying at least one thread 11 onto a substrate 12 intended to produce these sanitary articles.

By way of non-limiting example, reference will be made hereinafter to a single elastic thread 11 and to a substrate 12 that comprises at least one non-woven fabric material.

The substrate 12 can be a continuous web, a discrete element, or another component of a sanitary article. For example, the guiding device 10 allows application of an elastic thread 11 between two substrates 12 of flexible but not elastic fabric and to trap it between them so as to render the composite fabric elastic.

According to possible embodiments, the guiding device 10 comprises a first body 13 movable along a linear direction X and configured to guide the thread 11, and at least one second body 14 fixed with respect to the first body 13.

According to possible embodiments, the first body 13 is configured to carry a thread 11 supplied thereto, which is guided towards the substrate 12 by moving the first body 13 itself.

The guiding device 10 also comprises a magnetic movement assembly 15 associated with the first body and with the at least one second body for moving the first body along the linear direction by means of a magnetic force.

In accordance with an aspect of the present invention, the guiding device 10 comprises a spacer assembly 16 configured to space the first body 13 apart from the at least one second body 14 by means of an air cushion.

According to possible embodiments, thanks to the action of the spacer assembly 16, a gap 17 is formed between the first body 13 and the at least one second body 14, also considering any elements associated with them.

As will be clear below and as can be seen in the drawings, for example, the possible elements associated with the first body 13 and/or with the at least one second body 14 may comprise magnets 18, means 19 for generating a variable magnetic field, the aerostatic spacer elements 27, 127, or other elements.

According to possible embodiments, the minimum width of the gap 17 can be between 1 µm and 20 µm. According to possible embodiments, the minimum width of the gap 17 can be between 5 µm and 15 µm.

According to possible embodiments, the magnetic drive assembly 15 may comprise a plurality of magnets 18 and means 19 for generating a variable magnetic field so as to cooperate, in use, with at least part of the magnets 18 to move the first body 13 along the linear direction X.

This embodiment makes it possible to control the movement of the first body 13 through the means 19 for generating a variable magnetic field without the need to also intervene directly on the magnets 18, whether the magnets are configured to generate a variable magnetic field, or if they are electromagnets.

This greatly simplifies the operations of control and maintenance on the components, and basically limits any spare parts to just the means 19 for generating a variable magnetic field.

According to possible embodiments, the magnetic movement assembly 15 may comprise a plurality of magnets 18, placed on the first body 13, and means 19 for generating a variable magnetic field, placed on the at least one second body 14.

According to possible embodiments, the guiding device 10 may comprise at least one management and control unit 31 configured to modulate the variable magnetic field generated by the means 19, by means of a control signal sent by said management and control unit 31 to the means 19.

Thanks to the management and control unit 31 it is possible to make the guiding device 10 flexible, in other words able to move the first body 13 in accordance with the specific production requirements. This allows entrapment patterns of the threads 11 to be created in the substrates 12 and therefore enables production of various and different types of sanitary articles.

By modulating the variable magnetic field in the desired manner, the first body 13 performs various movements in relation to the sanitary article to be produced.

Thanks to this aspect, it is no longer necessary to completely replace the guiding device 10 for each type of sanitary article to be made.

Moreover, the possibility of also being able to modify the movement of the first body 13 during feeding of the thread 11 makes it possible to manage the pull of the thread in relation to the elasticity of the thread 11 and to the instantaneous position of the first body 13 along the linear direction X.

According to possible embodiments, modulating the variable magnetic field to modify - in real time - the movement of the first body 13 to compensate for the pulling effects of the thread 11 can be determined once the linear direction X has been defined with respect to the application point of the thread 11 on the substrate 12.

In accordance with possible embodiments, the magnets 18 can be permanent magnets such as, for example, magnets comprising iron, or other magnetic elements. For example, the magnets 18 may comprise neodymium, iron and boron which, when combined, exhibit high energy densities and operating temperatures.

According to possible embodiments, the means 19 for generating a variable magnetic field may comprise one or more reels possibly coupled to ferromagnetic cores which allow generation of a variable magnetic field in relation to the electric current that is supplied to them.

These reels can be spatially positioned so as to generate - each time - a magnetic field having the desired distribution of intensity and direction.

According to possible embodiments, the means 19 for generating a variable magnetic field can be magnetic actuators.

In relation to the specifications of the variable magnetic field generated by the means 19 and to the mutual position of the magnets 18 with respect to the means, the first body 13 is moved along the linear direction X.

According to possible embodiments, the plurality of magnets 18 can be placed on the first body 13 and the means 19 for generating a variable magnetic field can be placed on the at least one second body 14.

This aspect makes it possible to position the means 19 - which require a connection to power supply sources - on the at least one second fixed body 14, while the magnets 18, which do not require connections to further elements, are positioned on the first movable body 13.

This makes it possible to make the movable body 13 light and devoid of additional bulky or obstructing elements such as, for example, connections to the power supply sources, which make assembly and use complex.

According to possible embodiments, the magnets 18 can be placed in succession along the extension of the first body 13 which extends along the linear direction X.

In accordance with possible embodiments, the means 19 for generating a variable magnetic field can be placed on the at least one second body 14 so that they face the plurality of magnets 18.

By positioning the means 19 for generating a variable magnetic field on the at least one fixed second body 14, it is possible to size and position the connections towards the source of electrical power that supplies the means 19 so that they are stable and do not obstruct the movement of the first body 13. This aspect simplifies any maintenance operations on the guiding device 10.

According to possible embodiments, the first body 13 can be a profiled bar having an L-shaped cross-section defined by a long side 20 and a short side 21.

As will become clear later in the description, this conformation of the first body 13 allows the position of the first body to be stabilized with respect to that of the at least one second body 14 along both directions perpendicular with respect to the linear direction X.

In accordance with possible embodiments, the magnets 18 can be placed on the long side 20 of the profiled bar. For example, the magnets 18 can be placed in the central area of the long side 20 that extends along the linear direction X.

In this way, the short side 21 is not affected by the action of the means 19 on the magnets 18 and can advantageously be used to connect a thread-carrying tool, also referred to below as a support member 24.

In order to contain the temperature within the operating limits of the magnets 18 and of the means 19 without risk of damaging and/or deforming the first body 13, the at least one second body 14, or other, the guiding device 10 may comprise one or more insulating elements 22 associated with the magnets 18 and/or the means 19 for generating a variable magnetic field, so as to thermally isolate the first body 13 and the at least one second body 14 from the heat generated by the magnets 18 and/or by the means 19.

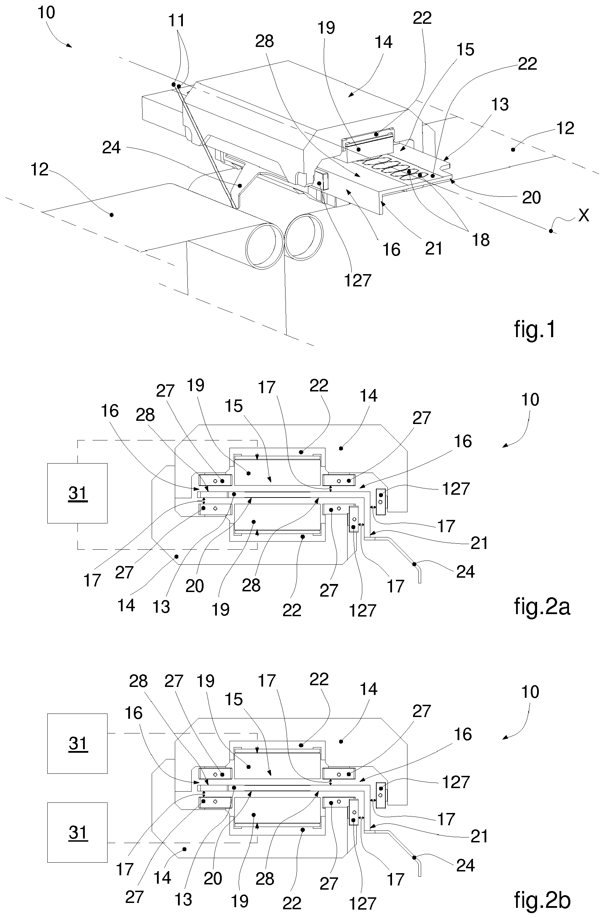

For example, the insulating elements 22 may comprise epoxy glass or other heat-insulating materials. With reference to Fig.1, the insulating element 22 can be placed between the magnets 18 and the first body 13 along the linear direction X and/or also between the means 19 and the second body 14.

According to possible embodiments, the guiding device 10 may comprise a cooling unit 23 configured to cool at least the first body 13 by means of a cooling fluid.

According to possible embodiments, the guiding device 10 may comprise a cooling unit 23 configured to cool at least the second body 14 by means of a cooling fluid.

In accordance with possible embodiments, the cooling unit 23 may comprise cooling ducts integrated or coupled to the second body 14 and fluidly connected to a cooling source which supplies a cooling fluid.

Still with the aim of maintaining the temperature within the operating values and guaranteeing a constant air circulation, the cooling unit 23 may comprise one or more fans configured to circulate air along the linear direction X.

In accordance with possible embodiments, the fans are configured to generate an air flow that circulates along the linear direction X and exits through the opening from which the support member 24 protrudes.

This aspect hinders the access of contaminants inside the guiding device 10 and, in particular, in the gaps 17 between the first body 13 and the second body 14.

According to possible embodiments, the means 19 for generating a variable magnetic field can be coupled to a cooling unit 23 configured to cool the means 19 and/or the at least one second body 14 to bring and maintain the temperature within the operating values.

In accordance with possible embodiments, the guiding device 10 may comprise at least two second bodies 14 between which the first body 13 is interposed, wherein each of the at least two second bodies 14 is provided with respective means for generating a respective variable magnetic field.

This aspect is particularly advantageous in that the combined action of the two second bodies 14 between which the first body 13 is interposed provides greater stability and uniformity of thrust to the first body 13.

The two second bodies 14 facing towards the first body 13 allow the balance of the attraction force and, furthermore, significant containment of the load on the aerostatic spacers 27, since the attraction force between the second bodies 14 and the first body 13 produces a pre-load useful for optimizing the action of aerostatic spacers 27.

According to possible embodiments, the guiding device 10 may comprise a single management and control unit 31 configured to modulate both variable magnetic fields generated by the respective means 19 of the at least two second bodies 14, by means of a control signal sent by the management and control unit 31 to the means 19.

According to possible embodiments, the guiding device 10 may comprise at least two management and control units 31, each configured to modulate the respective variable magnetic field generated by the respective means 19 of the at least two second bodies 14, by means of a respective control signal sent by the management and control unit 31 to the respective means 19.

According to possible embodiments, the magnets 18 are placed on both sides of the long side 20 of the first body 13 along the linear direction X, while the means 19 are placed on the two second bodies 14 facing towards a respective face of the long side 20 of the first body 13.

The combined action of the means 19 for generating a magnetic field on the magnets 18 allows the first body 13 to be maintained in the correct position, significantly reducing the load supported by the spacer assembly 16.

This solution allows the movement of the first body 13 to be sped up even more and, furthermore, allows reduction of the load on the second bodies 14 considered individually with the same thrust obtained with a single second body 14 provided with means 19 for generating a variable magnetic field.

The guiding device 10 of an apparatus for producing sanitary articles according to the present invention can reach speeds of movement of the first body 13 of up to 12 m/s with accelerations at full speed even higher than 230 m/s² and with strokes even greater than 30 cm.

The present invention has the considerable advantage that it allows generation of high energy which can be used to supply the guiding device 10 itself and/or other devices.

Unlike the prior art in which accelerations and decelerations can reach about 100-150 m/s , the combined and synergic action of the spacer assembly 16 and of the magnetic movement assembly 15 allow accelerations and decelerations of up to 250-350 m/s² to be reached.

The variations in reciprocal speed of the magnets 18 with respect to the means 19 and, in particular, the deceleration step allows generation of an electric current in the means 19 which can be conveyed towards the network and/or other components according to the specific requirements.

According to possible embodiments, the electrical energy generated during the deceleration step of the first body 13 on which the magnets 18 are arranged can be used to supply the spacer assembly 16 and/or the magnetic power supply assembly 15. This makes it possible to make the guiding device 10 at least partly self-powered.

According to possible embodiments, the ratio between the respective lengths along the linear direction X of the first body 13 and of the second body 14 can be greater than or equal to 1.

According to possible embodiments, the ratio between the respective lengths along the linear direction X of the first body 13 and of the second body 14 can be from 1 to 3.

According to possible embodiments, the ratio between the respective lengths along the linear direction X of the first body 13 and of the second body 14 can be from 1.5 to 2.5.

These geometric relationships between the first body 13 and the at least one second body 14 have been set up so as to balance and optimize the relationship between bulkiness and energy transfer efficiency.

These identified geometric ratios allow the totality of the magnetic field generated by the means 19 to be used for generating a variable magnetic field of the at least one second body 14 to move the first body 13.

In fact, it appears that the area of action of the means 19 on the at least one second body 14 faces, for its greater part, or for its totality, towards the area of the first body 13 where the magnets 18 are arranged.

This allows the first body 13 to be moved without high electromagnetic losses of the means 19 for generating a variable magnetic field, since the magnetic energy generated by these means is transferred to the first body 13.

In agreement with possible embodiments, the guiding device 10 may comprise a support member 24 connected to the first body 13 and configured to connect a tool to carry the thread 11, in which the support member extends, at least in part, out of the at least one second body 14.

This aspect makes it possible to position and guide the thread 11 in a region which is far from both the magnetic movement unit 15 and the spacer assembly 16. This is advantageous in that the thread 11, the tool and the support member 24 are not affected by the magnetic field or by the action of the spacer assembly 16.

According to possible embodiments, the support member 24 and/or the tool can simultaneously carry and guide a plurality of threads 11.

According to possible embodiments, the support member 24 can be fixed to the short side 21 of the first body 13 by means of suitable fastening elements such as, for example, pegs, welding joints, or the like. According to possible embodiments, the support member 24 can be made in a single piece with the first body 13.

In accordance with possible embodiments, the guiding device 10 may comprise a casing 25 configured to contain the first body 13, the at least one second body 14, the spacer assembly 16 and the magnetic movement assembly 15.

In order to limit exposure to contamination and make the guiding device 10 safe for operators, the casing 25 is provided with a slot 26 along which at least part of the support member 24 comes out and slides.

According to possible embodiments, the casing 25 may comprise a plurality of shells which can be connected to each other in a removable manner.

In accordance with possible embodiments, the connectors 30 assigned to the electrical connection or to the fluid connection, be it air, or with any other gas, as well as with a cooling fluid, can be arranged on the side faces of the casing 25 placed transversely with respect to the linear direction X.

According to possible embodiments, the spacer assembly may comprise a plurality of aerostatic spacer elements 27, 127 each configured to generate a respective air cushion in order to locally distance the first body 13 from the at least one second body 14.

This aspect allows distancing the first body 13 away from the at least one second body 14 in a desired manner and when required, also diversified, making the guiding device 10 flexible for use in multiple conditions and settings.

According to possible embodiments, the guiding device 10 may comprise a first body 13 and a single second body 14. For example, the first body 13 can be a profiled bar, or a profiled bar having an inverted-U-shaped cross-section.

In the step of actuating the aerostatic spacer elements 27, the magnetic attraction force between the means 19 and the magnets 18 allows the first body 13 to be retained in the correct position.

In the case of a single second body 14, the magnetic attraction force between the means 19 and the magnets 18 allows the first body 13 to be retained in the correct position, avoiding that, above all in the step of actuating the aerostatic spacer elements 27, the first body 13 is excessively distanced from the second body 14.

According to possible embodiments, for each second body 14 of the guiding device 10, the spacer assembly 16 may comprise at least one aerostatic spacer element 27 which acts to space the first body 13 apart from the respective second body 14 along a first direction perpendicular with respect to the linear direction X and at least one other aerostatic spacer element 127 which acts to space the first body 13 away from the respective second body 14 along a second direction perpendicular to the linear direction X.

According to possible embodiments, the first and the second perpendicular direction are perpendicular to each other.

For example, if the first body 13 is a profiled bar having an L-shaped cross-section and there are two second bodies 14, the spacer assembly 16 may comprise at least one pair of aerostatic spacer elements 27 configured to space apart the long side 20 from the two second bodies 14 along a first direction perpendicular to the surface 32 of the first body 13 affected by the magnetic movement assembly 15, or the surface 32 facing the means 19 for generating the variable magnetic field, or rather the surface 32 on which the magnets are arranged 18.

According to possible embodiments, the spacer assembly 16 may comprise a pair of aerostatic spacer elements 127 configured to space apart the short side 21 from the second bodies 14 along a second direction perpendicular to the first perpendicular direction.

This aspect allows the first body 13 to be stabilized and kept at a distance from the second bodies 14 along both directions perpendicular to the linear direction X.

It is understood that the considerations relating to the illustrative case of a first body 13 comprised between two second bodies 14 can be extended to the case of a single second body 14 by suitably adapting geometries and/or reciprocal positions of the first body 13, of the second body 14, as well as of the elements possibly associated with them.

In accordance with possible embodiments, the aerostatic spacer elements 27 can be placed on the at least one second body 14 at the sides of the magnetic movement assembly 15.

According to possible embodiments, the aerostatic spacer elements 27 can face towards the side portions 28 of the first body 13 between which the magnets 18 are arranged.

This aspect allows both stabilization of the positioning of the first body 13 with respect to the at least one second body 14, and separation of the area of action of the magnetic movement assembly 15 with respect to the areas of action of the aerostatic spacer elements 27.

The presence of the aerostatic spacer elements 27 on the at least one second body 14 at the sides of the magnetic movement assembly 15 allows positioning of the first body 13 on a stable and defined plane of arrangement, while ensuring continuity of operation without undesired interference between the spacer assembly 16 and the magnetic drive assembly 15.

According to possible embodiments, the side portions 28 can be provided with tracks comprising anti-friction material configured to reduce the friction between the first body 13 on the at least one second body 14 if there is contact between the first body and the second body. For example, the track may include a material called Turcite®, or other similar materials.

According to possible embodiments not illustrated, the spacer assembly 16 may comprise at least one magnetic spacer element configured to locally distance the first body 13 from the at least one second body 14 by means of another magnetic force.

According to possible embodiments, the magnetic spacer elements may comprise magnets and/or means for generating a magnetic field and are located on the sides of the magnetic movement assembly 15 so as not to hinder the action of its movement.

For example, the magnetic spacer elements can be substantially configured in a manner analogous to the magnetic movement assembly 15, but with the object of distancing the first body 13 from the at least one second body 14 instead of moving the first body 13 with respect to the at least one second body 14.

In accordance with possible embodiments, the magnetic spacer elements can be located either on the long side 20, or on the short side 21.

This aspect allows reduction of the energy consumption to generate the air cushion, since part of the energy necessary to distance the first body 13 from the at least one second body 14 is supplied by one or more magnetic spacer elements, also passively, or rather, by permanent magnets.

Thanks to the possibility of generating two dedicated magnetic fields, respectively, for distancing the bodies 13 and 14 and for reciprocally moving them, it is possible to simplify the guiding device 10, making positioning and movement precise, stable and reproducible.

In accordance with possible embodiments, the present invention also relates to a method using a guiding device 10 of an apparatus for producing sanitary articles as described in any one of the embodiments described for applying a thread 11 onto a substrate 12 intended for producing sanitary articles.

In accordance with one aspect of the present invention, the method envisages: associating the thread 11 with a first body 13 movable along a linear direction X; spacing the first body 13 apart from the at least one second body 14 that is fixed with respect to the first body 13, by means of an air cushion; and moving the first body 13 along the linear direction X by a magnetic force so as to guide the thread 11 in order to apply it onto the substrate 12.

According to possible embodiments, the movement of the first body 13 can be obtained by means of a magnetic movement assembly 15 comprising a plurality of magnets 18, placed on the first body 13, and means 19 for generating a variable magnetic field, placed on the at least one second body 14.

In accordance with possible embodiments, the method envisages generating electrical energy by electromagnetic induction, while the first body 13 decelerates, and feeding an apparatus with the generated electric energy.

This aspect makes it possible to generate electrical energy that can be used to supply parts of the guiding device 10 such as, for example, the spacer assembly 16, and/or other devices and/or apparatuses.

According to possible embodiments, the method may envisage setting a desired timetable defining the position of the first body 13 along the linear direction X in relation to time, and for moving the first body 13 according to the set timetable.

In accordance with possible embodiments, the method may envisage modulating the movement of the first body 13 in relation to the elasticity of the thread 11 and to the instantaneous position of the first body 13 along the linear direction X.

It is clear that modifications to the guiding device 10 of an apparatus for producing sanitary articles, and to the method for applying a thread 11 onto a substrate 12 intended for producing sanitary articles described up to now can be made and/or parts added, without thereby departing from the scope of the present invention.

It is also clear that, although the present invention has been described with reference to some specific examples, a person skilled in the art will certainly be able to produce many other equivalent forms of the guiding device 10 of an apparatus for producing sanitary articles, and of the method having the characteristics set forth in the claims and therefore all falling within the scope of protection defined therein.

In the following claims, the references in brackets have the sole object of facilitating the reading and should not be considered as limiting factors regarding the scope of protection underlying the specific claims.

## Claims

1. A guiding device of an apparatus for producing sanitary articles for applying at least one thread (11) onto a substrate (12) intended to produce said sanitary articles, comprising a first body (13) movable along a linear direction (X) and configured to guide said thread (11), at least one second body (14) fixed with respect to said first body (13), a magnetic movement assembly (15) associated with said first body (13) and with said at least one second body (14) for moving said first body (13) along said linear direction (X) by means of a magnetic force, wherein said guiding device is **characterized in that** it comprises a spacer assembly (16) configured to space said first body (13) apart from said at least one second body (14) by means of an air cushion, and **in that** said magnetic movement assembly (15) comprises a plurality of magnets (18), placed on said first body (13), and means (19) for generating a variable magnetic field, placed on said at least one second body (14), wherein said variable magnetic field is able to cooperate, in use, with at least part of said magnets (18) to move said first body (13) along said linear direction (X) by means of said magnetic force.

2. A device as in claim 1, **characterized in that** said spacer assembly (16) comprises a plurality of aerostatic spacer elements (27, 127) each configured to generate a respective air cushion in order to locally distance said first body (13) from said at least one second body (14).

3. A device as in claim 2, **characterized in that** said aerostatic spacer elements (27, 127) are placed on said at least one second body (14) at the sides of said magnetic movement assembly (15).

4. A device as in any one of the preceding claims, **characterized in that** said spacer assembly (16) comprises at least one magnetic spacer element configured for locally distancing said first body (13) from said at least one second body (14) by another magnetic force.

5. A device as in any of the preceding claims, **characterized in that** the ratio between the respective lengths along said linear direction (X) of said first body (13) and of said second body (14) is greater than 1.

6. A device as in any one of the preceding claims, **characterized in that** it comprises at least one management and control unit (31) configured to modulate said variable magnetic field generated by said means (19), by means of a control signal sent by said management and control unit (31) to said means (19).

7. A device as in any of the previous claims **characterized in that** it comprises at least two second bodies (14) between which said first body (13) is interposed, wherein each of said at least two second bodies (14) is provided with respective means (19) for generating a respective variable magnetic field.

8. A device as in claim 7, **characterized in that** it comprises at least two management and control units (31) each configured to modulate the respective variable magnetic field generated by the respective means (19) of said at least two second bodies (14), by means of a respective control signal sent by said management and control unit (31) to the respective means (19).

9. A device as in any one of the preceding claims, **characterized in that** it comprises a cooling unit (23) configured to cool at least said first body (13) by means of a cooling fluid.

10. A method for applying at least one thread (11) onto a substrate (12) intended for producing sanitary articles using a guiding device (10) as claimed in any one of the previous claims, wherein said method envisages: associating said thread (11) with a first body (13) movable along a linear direction (X); spacing said first body (13) apart from at least one second body (14) that is fixed with respect to said first body (13) by means of an air cushion; and moving said first body (13) along said linear direction (X) by means of a magnetic force so as to guide said thread (11) to apply it onto said substrate (12), wherein the movement of said first body (13) is obtained by means of a magnetic movement unit (15) comprising a plurality of magnets (18), placed on said first body (13), and means (19) for generating a variable magnetic field, placed on said at least one second body (14).

11. A method as in claim 10, **characterized in that** it envisages generation of electrical energy by electromagnetic induction, while said first body (13) decelerates, and feeding an apparatus with said generated electrical energy.

12. A method as in claim 10 or 11, **characterized in that** it envisages setting a desired timetable defining the position of said first body (13) along said linear direction (X) in relation to time, and to move said first body (13) in accordance with said set timetable.

13. A method as in any one of the claims from 10 to 12, **characterized in that** it envisages modulation of the movement of said first body (13) in relation to the elasticity of said thread (11) and the instantaneous position of said first body (13) along said linear direction (X).
